# EUROPEAN PATENT APPLICATION

(11) **EP 3 699 185 A1**
(43) Date of publication of application: **26.08.2020**
(21) Application number: 19305205.7
(22) Date of filing: 20.02.2019
(51) Int. Cl.: C07K 7/06, C07K 7/08, C07K 14/415

(54) **PEPTIDES FOR PREVENTING BIOFILM FORMATION**

(71) Applicant: Université de Rennes 1, 35065 Rennes Cedex (FR); CENTRE NATIONAL DE LA RECHERCHE SCIENTIFIQUE, 75016 Paris (FR); Universidade Federal Do Rio Grande Do Sul - UFRGS, 90046-900 Porto Alegre - RS (BR)
(72) Inventor: GOMES VON BOROWSKI, Rafael, 93520-632 Novo Hamburgo - RS (BR); RIGON ZIMMER, Aline, 90660-360 Porto Alegre,Rio Grande do Sul (BR); BAGGIO GNOATTO, Simone Cristina, Porto Alegre, RS Brasil CEP 90050240 (BR); MACEDO, Alexandre José, 90590-130 Porto Alegre (BR); PRIMON DE BARROS, Muriel, 90020-120 Porto Alegre (BR); RIGON ZIMMER, Karine, 90050-330 Porto Alegre (BR); GILLET, Reynald, 35160 Le Verger (FR); GOSMANN, Grace, 0610-000 Porto Alegre (BR)
(74) Representative: Lavoix

(57) **Abstract**

The present invention relates to the use of a peptide to prevent biofilm formation by microorganisms on a surface, wherein said peptide is
(i) a peptide consisting of 10 to 37 consecutive amino acids from a peptide of sequence SEQ ID NOS: 1,
(ii) a peptide of sequence SEQ ID NO: 4,or
(iii) a peptidomimetic of (i) or (ii).

SEQUENCE LISTING

<110>  Federal University of Rio Grande do Sul
       Universite Rennes 1
       CNRS

<120>  Peptides for preventing biofilm formation

<130>  BET 18P4296

<160>  6

<170>  PatentIn version 3.5

<210>  1
<211>  37
<212>  PRT
<213>  Artificial Sequence

<220>
<223>  P31

<400>  1

Arg Ser Cys Gln Gln Gln Ile Gln Gln Ala Gln Gln Leu Ser Ser Cys
1               5                   10                  15

Gln Gln Tyr Leu Lys Gln Arg Val Gln Ser Glu Glu Gly Glu Asp Gln
            20                  25                  30

Ile Ser Gln Arg Glu
        35

<210>  2
<211>  22
<212>  PRT
<213>  Artificial Sequence

<220>
<223>  P3

<400>  2

Arg Ser Cys Gln Gln Gln Ile Gln Gln Ala Gln Gln Leu Ser Ser Cys
1               5                   10                  15

Gln Gln Tyr Leu Lys Gln
            20

<210>  3
<211>  15
<212>  PRT
<213>  Artificial Sequence

<220>
<223>  P1

<400>  3

Arg Val Gln Ser Glu Glu Gly Glu Asp Gln Ile Ser Gln Arg Glu
1               5                   10                  15

<210>  4
<211>  17
<212>  PRT
<213>  Artificial Sequence

<220>
<223>  P2

<400>  4

Arg Ala Glu Ala Phe Gln Thr Ala Gln Ala Leu Pro Gly Leu Cys Arg
1               5                   10                  15

Ile

<210>  5
<211>  10
<212>  PRT
<213>  Artificial Sequence

<220>
<223>  P3b

<400>  5

Leu Ser Ser Cys Gln Gln Tyr Leu Lys Gln
1               5                   10

<210>  6
<211>  12
<212>  PRT
<213>  Artificial Sequence

<220>
<223>  P3a

<400>  6

Arg Ser Cys Gln Gln Gln Ile Gln Gln Ala Gln Gln
1               5                   10

## Description

The present invention relates to peptides for preventing biofilm formation by microorganisms.

Antimicrobial failure is a worldwide challenge, endorsed in a currently global action plan. The lack of novel antibiotics and their inappropriate uses are resulting in an increase of multi-drug tolerant and resistant strains. This process is favored by biofilm development hence microorganisms enclosed in the matrix display up to 1000 times higher antibiotic resistance than the planktonic ones making the biofilm matrix itself a new important target.

Moreover, the biofilm is a complex matrix, composed of extracellular polymeric substance (EPS) wrapping microorganisms communities, irreversibly adhered to a biotic (e.g. tissues and organs) or abiotic (e.g. catheters, prostheses, kitchen or marine utensils) surface. Micro colonies are the beginning of mature biofilm and its structure and composition can react or be adsorbed with external agents mediating the adhesion to surfaces and also promoting physical protection against the antibiotics or the immune system response. Accordingly, adhesion and colonization are required for the establishment of bacterial infection and pathogenesis. Thus, the bacteria may undergo specific molecular changes to establish biofilms and adhering on implanted devices as probes, prostheses, catheters or on damaged tissues, impacting importantly on the patients' denouement and costs of the health system.

In this context, *Staphylococcus epidermidis* is the most frequently negative coagulase *Staphylococcus* infection causing disease, being capable to survive on surfaces for months. *Staphylococcus epidermidis* is an emerging pathogen bacteria and the ability to form biofilms on devices is its major virulence factor. Biofilm has been considered an important virulence factor, which is present in 80% of human infections as endocarditis, osteomyelitis, chronic sinusitis, urethritis, periodontitis, characterizing it as a severe public health problem. The extracellular matrix is a complex physicochemical barrier that represents one of the highest difficulties in prevention or treatment of biofilm. Biofilms are often the cause of difficulty in eradicating bacteria, representing a challenge in different areas, especially medical, odontological, navy and food industry surroundings.

Therefore, the development of antivirulence strategies such as efficient antibiofilm agents is crucial against the current antibiotic crisis.

In particular, there is a need for identifying antiadhesive agents as an alternative strategy to prevent bacterial attaching and infection.

Currently, there is a great interest in the search for new natural bioactive compounds against bacterial biofilms, ubiquitous in natural, clinical and industrial environments, and responsible for the high tolerance and resistance of bacteria to antimicrobial agents. Some non-biocidal strategies that target the related virulence factors have been proposed. In this scenario, peptides and peptidomimetics are rising as important arsenal. However, there are currently no anti-biofilm drugs available.

Thus there is still a need for anti-biofilm strategies targeting biofilm matrix in order to fight multi-drug tolerant and resistant bacteria.

Plant-derived compounds have gained extensive interest in the search for alternatives to control microorganisms. These compounds are widely accepted due to their use in folk medicine for the prevention and treatment of diseases and infections.

The extracts from fruits of *Capsicum baccatum* var. *pendulum,* a pepper widely consumed in Brazil, have been shown to present antioxidant, anti-inflammatory, and antifungal properties. However, there is still scare biological and chemical information about this species.

The present invention arises from the unexpected finding by the inventors that peptides isolated from an extract of *C. baccatum* seeds have potent anti-biofilm activity. More specifically, the inventors have demonstrated herein that peptides derived from 2S sulfur-rich seed storage protein (s.s.p) 2-like from *C. baccatum* significantly inhibits biofilm formation by microorganisms.

In particular, the inventors have discovered new antibiofilm peptides derived from the red pepper *Capsicum baccatum* that prevent adhesion, biofilm establishment and maintenance of microorganisms. Importantly, these peptides are non-antibiotic and non-cytotoxic, providing a new alternative to prevent biofilm infections. In particular, the peptides according to the invention prevent bacterial adhesion and biofilm formation of the methicillin resistant *S*. *epidermis* and do not show antibiotic activity. These properties evidence their potential for combating antibiotic-tolerant and resistant bacteria, and controlling clinical and industrial problems related to biofilms.

The present invention thus relates to the use of a peptide to prevent biofilm formation by microorganisms on a surface, wherein said peptide is:
(i) a peptide consisting of a fragment of 10 to 37 consecutive amino acids residues from a peptide of sequence SEQ ID NO: 1 (RSCQQQIQQAQQLSSCQQYLKQRVQSEEGEDQISQRE),
(ii) a peptide of sequence SEQ ID NO: 4 (RAEAFQTAQALPGLCRI), or
(iii) a peptidomimetic of (i) or (ii).

It also relates to an isolated peptide consisting of an amino acid sequence selected from the group consisting of:
SEQ ID NO: 2 (RSCQQQIQQAQQLSSCQQYLKQ),
SEQ ID NO: 1 (RSCQQQIQQAQQLSSCQQYLKQRVQSEEGEDQISQRE),
SEQ ID NO: 3 (RVQSEEGEDQISQRE),
SEQ ID NO: 4 (RAEAFQTAQALPGLCRI),
SEQ ID NO: 5 (LSSCQQYLKQ), and
SEQ ID NO: 6 (RSCQQQIQQAQQ),
or a peptidomimetic thereof.

The present invention also relates to a composition comprising the isolated peptide of the present invention.

The present invention also relates to an *ex vivo* method for preventing biofilm formation by microorganisms on a device comprising at least one surface, said method comprising the step of coating said at least one surface with the composition of the present invention.

The present invention further relates to a device comprising at least one surface coated with the peptide of the present invention.

The present invention also relates to the peptide of the present invention for use in a method for preventing infections such as endocarditis, osteomyelitis, chronic sinusitis, urethritis or periodontitis in a patient by the prevention of biofilm formation by microorganisms.

### Detailed description of the invention

### Peptide

The terms "peptide" refers to amino acid sequences of a variety of lengths. In preferred embodiments, the amino acid sequence is a fragment of the peptide of the present invention.

In a particular embodiment, the peptide according to the invention is a peptide consisting of an amino acid sequence selected from the group consisting of:
- SEQ ID NO: 2 (RSCQQQIQQAQQLSSCQQYLKQ),
- SEQ ID NO: 1 (RSCQQQIQQAQQLSSCQQYLKQRVQSEEGEDQISQRE),
- SEQ ID NO: 4 (RAEAFQTAQALPGLCRI)
- SEQ ID NO: 3 (RVQSEEGEDQISQRE),
- SEQ ID NO: 5 (LSSCQQYLKQ), and
- SEQ ID NO: 6 (RSCQQQIQQAQQ),
or is a peptidomimetic thereof.

In a preferred embodiment, the peptide according to the invention is a peptide consisting of SEQ ID NO: 2.

The term "fragment", when used herein, refers to a peptide comprising or consisting of an amino acid sequence of at least 10 consecutive amino acid residues and of less than 38 consecutive amino acid residues of the peptide of sequence SEQ ID NO: 1, typically of 10, 11, 12, 13, 14, 15, 16, 17, 18, 19, 20, 21, 22, 23, 24, 25, 26, 27, 28, 29, 30, 31, 32, 33, 34, 35, 36, or 37 consecutive amino acids residues of the peptide of sequence SEQ ID NO: 1. In a particular embodiment, the peptide of the present invention is a fragment of 10, 11, 12, 13, 14, 15, 16, 17, 18, 19, 20, 21 or 22 consecutive amino acids residues from a peptide of sequence SEQ ID NO: 1, preferably 10, 12, 15 or 22 consecutive amino acid residues of the protein or polypeptide.

The invention also relates to peptidomimetics of the peptides according to the invention. Peptidomimetics refer to synthetic chemical compounds, which have substantially the same structural and/or functional characteristics of the peptides according to the invention. The mimetic can be entirely composed of synthetic, non-natural amino acid analogs, or can be a chimeric molecule including one or more natural amino acids and one or more non-natural amino acid analogs. The mimetic can also incorporate any number of natural amino acid conservative substitutions that do not destroy the mimetic's activity. The phrase "substantially the same", when used in reference to a mimetic or peptidomimetic, means that the mimetic or the peptidomimetic has one or more activities or functions of the referenced molecule, in particular prevention of biofilm formation. The techniques for developing peptidomimetics are conventional. For example, peptide bonds can be replaced by non-peptide bonds or non-natural amino acids that allow the peptidomimetic to adopt a similar structure, and therefore biological activity, to the original peptide. Further modifications can also be performed by replacing chemical groups of the amino acids with other chemical groups of similar structure. Once a potential peptidomimetic compound is identified, it may be synthesized and its ability to prevent biofilm formation can be assayed. Peptidomimetics can contain any combination of non-natural structural components, which are typically from three structural groups: residue linkage groups other than the natural amine bond ("peptide bond") linkages; non-natural residues in place of naturally occurring amino acid residues; residues which induce secondary structural mimicry (e.g., beta turn, gamma turn, beta sheet, alpha helix conformation); or other changes which confer resistance to proteolysis.

One or more residues can also be replaced by an amino acid (or peptidomimetic residue) of the opposite chirality. Thus, any amino acid naturally occurring in the L-configuration (which can also be referred to as R or S, depending upon the structure of the chemical entity) can be replaced with the same amino acid or a mimetic, but of the opposite chirality, referred to as the D-amino acid, but which can additionally be referred to as the R- or S-form.

In a particular embodiment, the peptidomimetic according to the invention is an AApeptide. AApeptides refers herein to oligomers of *N*-acylated-*N*-aminoethyl-substituted amino acids that are derived from chiral peptide nucleic acid backbones. The chiral side chain is connected to either the α-C or γ-C of the carbonyl group, while acylation is used to introduce the other side chain to the central N. AApeptides have the same backbone lengths and functional group counts, and the same number of nitrogen atoms involved in secondary or tertiary amide bonds than their original peptide counterparts. In addition, they mimic the original amino acid side-chain positions, so they have the same activity.

In another embodiment, the peptidomimetic according to the invention is a peptoid. Peptoids or beta-peptoids are oligomers of *N-*substituted glycine units. Their side chains extend from the main chain nitrogen rather than from the α-carbon, thus yielding secondary structures including helices, loops and turns. They retain the functionalities and backbone polarity of peptides.

In a particular embodiment, the peptidomimetic according to the invention is a peptidomimetic of a fragment of the peptide of the present invention.

The peptides and peptidomimetics can be produced and isolated from natural products using any method known in the art. Peptides can also be synthesized, whole or in part, using usual chemical methods.

### Biofilm

The inventors have shown that the peptide according to the invention prevent biofilm formation by microorganisms on a surface.

Microbial biofilms are ubiquitous in natural, clinical and industrial environments. Biofilms are complex communities of microorganisms, usually attached to a biotic and/or abiotic surface and encapsulated by a polymeric extracellular matrix of microbial origin. This complicated structure is involved in a multitude of different infections and contribute significantly to the therapeutic failures.

In the sense of the invention, the prevention of biofilm formation by microorganisms refers to the prevention of the establishment and maintenance of biofilm architecture. According to one alternative embodiment, the peptide according to the invention acts on the initial phase of matrix assembly, preventing its functional assembly rather than de-structuring once established. In another embodiment, the peptide interacts with the extracellular matrix and modifies the self-assembly chain, resulting in a less dense nonfunctional matrix that consequently prevents biofilm formation.

In the sense of the present invention, the microorganisms can be any microscopic organisms capable of forming a biofilm, for example bacteria or fungus. In a particular embodiment, the microorganisms may be *Staphylococcus epidermidis* (*S. epidermis*), *Pseudomonas aeruginosa (P. aeruginosa*) or *Cryptococcus neoformans (C. neoformans)*.

In a particular embodiment, the peptide according to the invention prevents biofilm formation without decreasing microorganisms' growth. In the sense of the invention the prevention of the biofilm formation is independent of cell death or growth inhibition. Microorganisms forming the biofilm are typically enclosed in the biofilm matrix and attached to a surface. They express higher physiological and biochemical changes and higher mutation rates than their planktonic forms. In the sense of the invention, the planktonic form of the microorganism refers to its non-adherent form and/or free flowing in suspension.

In a particular embodiment, the peptide according to the present invention does not inhibit planktonic growth of the biofilm-forming organisms. By the inhibition of the planktonic growth is meant the decrease or the end of the planktonic microorganism's growth rate.

Non-antibiotic targets suggest less susceptibility to the development of resistance phenomena than conventional antibiotics because microorganisms suffer a milder evolutionary pressure to generate resistance without the biotic activity. In another embodiment, the peptide according to the present invention prevents biofilm formation and limits the development of microorganisms that are drug resistant and/or tolerant.

In the sense of the invention, drug resistant microorganisms are microorganisms that survive to antimicrobial drugs, e.g. antibiotics, exposure by the acquisition of molecular resistance mechanisms. Drug tolerant microorganisms are microorganisms that survive antimicrobial drugs, e.g. antibiotics, exposure in the absence of acquired molecular resistance mechanisms.

In a particular embodiment, the microorganisms are resistant and/or tolerant to antimicrobial drugs.

In another embodiment, the microorganisms are antibiotic tolerant and/or resistant.

### Surface

In the sense of the invention, the surface on which the biofilm is formed can be any type of surface. The surface may be abiotic or biotic.

Biofilms are often the cause of difficulty in eradicating bacteria, representing a challenge in different areas, especially medical, odontological, navy and food industry surroundings.

The abiotic surface according to the invention may be the surface of a device or of a tooth, or an industrial processing surface. In a particular embodiment, the surface is an immersed surface, preferably a ship's hull, steel, metal, glass, polymers, minerals or ceramic material.

The biotic surface may be host tissue, mucus or a wound.

The present invention also relates a device comprising at least one surface as defined above coated with the peptide according to the invention. The device can be any industrial or medical device as defined above.

According to one advantageous embodiment of the invention, the surface according to the invention is the surface of a medical device.

The medical device according to the invention may be an implanted device or a surgical implant such as probes, prostheses, catheters. In particular, the medical device may be, but is not limited to, a ventricular derivation, an oro-tracheal tubing, a prosthetic cardiac valve, a pacemaker, an urinary catheter or an orthopedic prosthesis. In one particular embodiment, the medical device according to the invention is prone to the formation of biofilm and may lead to a microbial infection. Bacteria are a major concern for people with catheters, probes or other surgical implants because it is known to form biofilms on these devices.

According to another embodiment, the surface according to the invention is the surface of an industrial device.

The industrial device according to the invention may be a food or naval industry related device such as tools, equipments or instruments that have contact with water such as a pipe or a valve.

### Composition

The present invention also relates to a composition comprising the isolated peptide according to the present invention. In particular the isolated peptide may be the peptide consisting of SEQ ID NO: 2.

Accordingly, the present invention provides a composition comprising an effective amount of the peptide according to the invention. In a particular embodiment the concentration of the peptide in the composition is comprised between 1 and 100µM, in particular between 1 and 20µM, preferably between 1 and 10µM. In a preferred embodiment the concentration of the peptide in the composition is 10µM. In another embodiment, the composition according to the invention is effective from 1µM.

According to one advantageous embodiment, the composition may be a pharmaceutical composition. The pharmaceutical composition may comprise a pharmaceutically acceptable excipient.

The term "pharmaceutically acceptable" refers to properties and/or substances which are acceptable for administration to a subject from a pharmacological or toxicological point of view.

A pharmaceutical composition according to the invention may be administered in any amount and using any route of administration effective for achieving the desired prophylactic and/or therapeutic effect. The optimal pharmaceutical formulation can be varied depending upon the route of administration and desired dosage. Such formulations may influence the physical state, stability, rate of *in vivo* release, and rate of *in vivo* clearance of the administered active ingredient(s).

According to one advantageous embodiment of the invention, the pharmaceutical composition is administrated orally or intravenously. The pharmaceutical composition used within the scope of the invention may be presented in any dosage forms normally used for the oral or intravenous mode of administration. In a particular embodiment, the pharmaceutical composition used within the scope of the invention may be administrated in combination with antibiotics for the prevention and/or treatment of bacterial biofilm-associated infections.

Thus the present invention also relates to the peptide according to the invention for use for the prevention of biofilm formation by microorganism in a patient.

The present invention also relates to a method for preventing biofilm formation by microorganisms or infections in a patient in need thereof, said method comprising administering a prophylactically efficient amount of a peptide of the invention or of a composition of the invention to said patient.

The present invention also relates to the use of a peptide of the invention for the manufacture of a medicament intended to prevent infections in a patient.

In particular, the peptide according to the infection may be used for the prevention of infections linked to biofilm in a patient. In a particular embodiment, the infection may be endocarditis, osteomyelitis, chronic sinusitis, an urinary tract infection such as urethritis or an oral infection such as periodontitis.

By "prevention" is meant herein to prevent or slow down the emergence of an infection in a patient.

By "prophylactically efficient amount" is meant herein an amount effective, at dosages and for periods of time necessary, to achieve the desired prophylactic result.

The composition according to the invention may further be used to coat a surface as defined above and thus prevent the formation of a biofilm on said surface.

In a particular embodiment, the composition is an antifouling composition.

By antifouling composition is meant a composition which prevents biofouling, i.e the accumulation of microorganisms on wetted surface.

The present invention also relates to an *ex vivo* method for preventing biofilm formation by microorganisms on a surface as defined above, said method comprising the step of coating or modificating said surface with a composition according to the present invention. The coating or modification step may be carried out by any physical, chemical or depositional technique well-known from the skilled person, such as physical or thermal vapor deposition, solution-based processes, polymer-interlayers, biomimicry, induction hardening, nitriding, tufftriding and shot-peening,

The surface may be any type of surface according to the invention. In one embodiment, the method is an *ex vivo* method for preventing biofilm formation by a microorganisms on a device according to the present invention. In a particular embodiment, the method is an *ex vivo* method for preventing biofilm formation by a microorganisms on a medical or industrial device according to the present invention.

The present invention will be further illustrated by the examples below.

### Examples

### Materials and methods used in the examples

**Peptides.** All peptides were synthesized by Biomatik™ and ProteoGenix™ both containing a purity grade greater than 95% in salt suitable for cell culture. Mass spectral and HPLC analysis were provided as quality control. They were all solubilized in ultra-pure sterile water for the assays.

**Bacterial Strain and growth conditions.** *Staphylococcus epidermidis* ATCC 35984 was grown overnight on blood agar (Thermo Scientific, Oxoid PB5039A) at 37°C. A bacterial suspension of 3×10⁸ colony-forming units (CFU)/mL in tryptone soya broth (TSB, Oxoid Ltd., England, UK) or 0.9% NaCl was used in the assays. Lysogenic broth (LB, Oxoid Ltd., England, UK) agar was used to colony forming units (CFU) assay.

**Biofilm formation.** All assays were at least performed as technical and biological triplicates using 1, 10 or 100 µM of peptides. *Biofilm inhibition:* a protocol adapted from Zimmer and collaborators (Environmental Microbiology 2013, v.15 p.2008-2018) Trentin et al. (Scientific Reports 2015, v.5 p.8287) employing crystal violet in 96-well poly(vinyl chloride) microtiter plates (Falcon; Becton Dickinson Labware, Oxnard, CA) was used. Briefly, 100µL of the bacterial suspension, 100µL of the peptide solution (at different concentrations) or vehicle (to controls) and 50µL of tryptone soya broth (TSB, Oxoid Ltd., England). Following 37 °C for 1, 4 or 24 h of incubation, the content of the wells was removed and the wells were washed three times with sterile saline. The remaining contents were heat-fixed at 60 °C for 1 h. The adherent biofilm layer formed was stained with 0.4% crystal violet for 15 min at room temperature and then washed three times with distilled water. The stain bound to the cells was solubilized with absolute ethanol (Sigma-Aldrich Co., USA) and absorbance was measured at 570 nm (Powerwave™ XS Plate Reader, BIO-TEK instruments®, Inc.). The biofilm formation controls represent 100% of biofilm formation. *Biofilm eradication:* biofilm was pre-formed as described before, during 24 h at 37°C, without treatment. After biofilm formation, the wells were washed to remove the planktonic cells and the peptides solutions and controls were added and incubated for 24h. The eradication was verified by evaluating the remaining content by crystal violet as previous described.

**Bacterial growth assays.** *Microtiter plates:* bacterial growth was evaluated by difference between the optical density absorbance at 600nm measured at the end and the beginning of the incubation time (37 °C, 1, 4 or 24 h) in 96-well poly(vinyl chloride) microtiter plates. Rifampicin 16µg/mL (Sigma-Aldrich Co., USA) was used as a control for bacterial growth inhibition. *Colony-forming units (CFU*/*mL):* after incubation (37 °C, 24 h) the CFU was calculated to determine bactericidal effect of peptide solution. Untreated growth control was considered 100% of planktonic cells. All assays were at least performed as technical and biological triplicates.

**Scanning electron microscopy (SEM):** sterile polystyrene coupons (10 X 4mm) were co-culture in presence or absence of capsicumicine for 1, 4 and 24 hours. After, the coupons were washed with sterile NaCl 0.9% and fixed with glutaraldehyde 2.5%, paraformaldehyde 2%, cacodylate 0,1M buffer (pH 7.2). Afterwards, they were washed with cacodylate 0,1M buffer with sucrose 0.2 M and dehydrated with increasing concentrations of ethanol and dehydrated samples were then subjected to Critical Point Drying (Leica EM CPD 300). Finally, they were sputtered with palladium (Leica EM ACE 200) and analyzed by JEOL JSM 7100 F EDS EBSD Oxford microscope, at 10 kV.

**Transmission electron microscopy (TEM):** all the content of the wells was suitable detached (1, 4 and 24 hours cultures in presence or absence of P3), recovered, centrifuged at 10,000 g, 15 minutes, 4°C and washed with sterile NaCl 0.9%. Fixation was performed at 4°C with sodium cacodylate 0,1M, paraformaldehyde 2%, glutaraldehyde 2,5% and lysine 75mM. After that, samples were washed with sodium cacodylate 0,1M, sucrose 0,2M and contrasted with osmium tetroxide 1%, potassium ferrocyanide 1,5%. Dehydration was done with gradual solution of ethanol and infiltration with increasing concentration of LR White® resin (Delta Microscopies). Then, LR White® resin inclusion and polymerization were made during 24h at 60°C in O2 absence. Thin sections (80 nm) were collected onto 200 mesh carbon grids, and visualized with a Tecnai Sphera operating at 200 kV (FEI, Eindhoven, Netherlands) equipped with a 4x4 k CCD UltraScan camera (Gatan, Pleasanton, USA).

**Confocal fluorescence microscopy (CFM):** P3-fluorescein isothiocyanate (capsicumicine -FITC, 10µM) was used to detect capsicumicine peptide. After incubation (1, 4 and 24 hours) all the content of the wells was suitable detached, recovered, centrifuged at 11,000 g, 2 minutes, 4°C and washed with sterile NaCl 0.9%. This suspension was visualized directly or after Calcofluor 2mg/mL (Fluorescent Brightener 28, Sigma) addition. To illustrate bacterial cells permeable by a peptide (control) we used an antimicrobial peptide also labeled with FITC. Those antimicrobial peptides are seen on/in bacteria although not in the extracellular matrix. Images were acquired with Leica SP8 DMI 6000 CS (resonant scanner) confocal microscope with hybrid detector. ImageJ software was used for image analysis.

**Quantitative Reverse Transcriptase PCR (qRT-PCR):** the RNAs were isolated from planktonic cells (control), biofilm cells (control) or total cells (exposed to capsicumicine at 10µM), after 4, 24 hours cultures. It was applied TRIzol™ Max™ Bacterial RNA Isolation Kit (Invitrogen™) and TURBO™ DNase treatment (Ambion®) according to manufacturer's instructions. Concentration and purity of total RNA was spectrophotometrically assessed using SimpliNano™ (Biochrom, USA) and PCR reaction was performed to certify the complete absence of DNA. It was considered 50% of yield for Reverse Transcriptase Reaction (M-MLV, Promega®): 1000ng of RNA = 500ng cDNA. Then, we used 10ng of cDNA and 0.2µM of primers per qRT-PCR reaction, previously verified. Reactional volumes were calculated according to the manufacturer's instructions (SYBR® Select Master Mix, Applied Biosystems Inc; USA). Primers (Supplementary, Table 1) were designed through the Primer3 program (Thermo Fisher® Primers) and according to literature. They were produced by Eurofins Genomic. It was used Applied Biosystems StepOnePlus™ equipment and software. The relative transcript levels were determined by 2^{-ΔΔct} (Livak e Schmittgen, 2001). To validate the selected biofilm encoding genes we compared planktonic control to biofilm control. We found purposeful differences between biofilm and planktonic controls, as expected.

**Real time molecular self-assembly (RTMSA) assay:** after checking the starting point (pH) of the assembly reaction for staphylococcal synthetic matrix (Stewart et al., 2015, Sci Rep, v.5, p.13081), we recorded the optical density (OD = 600nm) in function of the time, each 30 seconds until 30 minutes. Molecular self-assembly reactions were calculated to a final volume of 4mL, considering 0,3% chitosan (medium molecular weight, 75-85% of deacetylation, Sigma), 0,15% bovine serum albumin (BSA, Sigma), 0,015% lambda DNA (Sigma) in tryptone soya broth (TSB, Oxoid Ltd., England, UK). The concentration of tested peptides was calculated in µM to the final volume of 4mL (100 µM). Before get the pH starting point of assembly reaction, a calibration record was done using the same reactional tube containing all reagents (auto zero). The pH adjustments were made using acetic acid and NaOH and the reaction temperature was around 30°C.

**Cytotoxicity assay:** the assays were performed in a robotic platform (ImPACcell, BIOSIT, Université de Rennes 1) dedicated to multiparameter high-throughput image analysis (HCS: High Content Screening and HCA: High Content Analysis), using 7 different mammalian lines: HuH7, CaCo-2, MDA, HCT116, PC3, NCI-H727 and MCF7. The concentration tested was 10 µM for peptide P3 (SEQ ID NO: 2) and 25 µM for peptide P31 (SEQ ID NO: 1). The number of normal cells is presented as residual cells percentage (%) compared to the average of DMSO control. Whereas, 100% represent no cytotoxicity or inhibition of cell growth, below 25/30% is considered cytotoxic and 0% represents acute cytotoxicity. This platform are equipped with Olympus right microscope (Spot NB camera and Simple PCI software, Compix), Right Zeiss Axiolmager M1 microscope (Marzhauser, Zeiss NB camera and AxioVision software) and the robots Arrayscan VTI Cellomics /Thermofisher, Hamilton Starlet, Hamilton Nimbus and Spotter Scienion.

### Example 1: Antibiofilm activity of the peptides

This example demonstrates the prevention of biofilm formation by *S. epidermidis* ATTCC 35984.

The inventors demonstrated that the peptides of the present invention interfere with biofilm formation. The peptides at 1, 10, 25 or 100µM were exposed to strong biofilm forming *S. epidermidis* RP62A (ATCC 35984). The remaining biofilm were quantified after 24 hours using crystal violet method. Biofilm decrease was observed at all tested concentrations. In particular, the peptide consisting of SEQ ID NO: 2 presented strong antibiofilm activity from 1 µM and more than 90% of biofilm reduction was detected at 10 µM.

They further demonstrated, surprisingly, that biofilm inhibition is not due to bactericidal activity. Accordingly, *S. epidermidis* colony forming units (CFU) was not affected in the presence of the peptide of SEQ ID NO: 2. Furthermore, the peptide has been localized by confocal fluorescence microscopy (CFM) images. The CFM images analysis show that the peptide does enter neither into bacterial cell nor into the wall or membrane but remains associated with extracellular matrix components.

In order to ensure the peptide future safe applicability, the inventors verified the biological cytotoxicity in 7 different mammalian lines (HuH7, CaCo-2, MDA, HCT116, PC3, NCI-H727 and MCF7), applying automated system image-based cellular content analysis (HCS / HCA). Thereby, cells treated with the peptide had exactly the same performance as untreated controls, evidencing absence of cytotoxicity.

As such, these results demonstrate that the peptides according to the invention may be used very effectively for preventing biofilm formation on a surface and acknowledge its safety by evidencing absence of cytotoxicity.

### Example 2: Impairment of initial attachment, aggregation and biofilm accumulation.

To reveal the peptide activity along the first stages of biofilm development, polystyrene coupons have been analyzed after 1, 4 and 24 hours of biofilm culture in presence or absence of the peptide. Scanning electron microscopy (SEM) analysis show bacteria attachment decreases after 1-hour of peptide exposition. Additionally, biofilm accumulation and cell aggregation profiles are strongly reduced after 4 and 24-hours demonstrating that the peptide prevents *S. epidermidis* coupons adherence. Notably this action remains after 24 h of incubation. The inventors further demonstrated, surprisingly, that antibiofilm mechanism of action is linked to extra cellular interactions independently of cell pressure regulation. The inventors selected some genes involved in different stages of biofilm development (*atlE, aap, agrC, icaA, leuA, saeR, saeS, sarA, gyrB, rrsA)* and analyzed its fold change by quantitative real-time PCR (qRT-PCR). Since exposed bacteria remain planktonic, the relative gene expression of them was compared to planktonic control cells. Exposed cells showed the same fold change as the planktonic control cells for all tested genes.

## Claims

1. Use of a peptide to prevent biofilm formation by microorganisms on a surface, wherein said peptide is
(i) a peptide consisting of 10 to 37 consecutive amino acids from a peptide of sequence SEQ ID NOS: 1,
(ii) a peptide of sequence SEQ ID NO: 4,or
(iii) a peptidomimetic of (i) or (ii).

2. Use according to claim 1, wherein said peptide is a peptide consisting of an amino acid sequence selected from the group consisting of SEQ ID NOS: 2 and 3.

3. Use according to claim 1 or 2, wherein said peptide does not inhibit planktonic growth of the biofilm-forming microorganisms.

4. Use according to claim 3, wherein said microorganisms are antibiotic tolerant or antibiotic resistant bacteria.

5. Use according to claim 3 or 4, wherein said microorganisms are selected from the group consisting of *Staphylococcus epidermis, Pseudomonas aeruginosa* and *Cryptococcus neoformans.*

6. Use according to any one of claims 1 to 5, wherein said surface is an abiotic or biotic surface.

7. Isolated peptide consisting of an amino acid sequence selected from the group consisting of SEQ ID NOS: 2, 1, 3 and 4, or a peptidomimetic thereof.

8. A composition comprising the isolated peptide of claim 7.

9. The composition according to claim 8 wherein said peptide consists of an amino acid sequence which is SEQ ID NO: 2.

10. The composition according to claim 9, wherein the concentration of said peptide in the composition is 1-10µM.

11. *Ex vivo* method for preventing biofilm formation by microorganisms on a device comprising at least one surface, said method comprising the step of coating said at least one surface with a composition according to any one of claims 8 to 10.

12. The method according to claim 11, wherein said device is a medical or industrial device.

13. A device comprising at least one surface coated with the peptide as defined in claim 1 or 2.

14. The peptide as defined in claim 7 for use in a method for preventing infections such as endocarditis, osteomyelitis, chronic sinusitis, urethritis or periodontitis in a patient by the prevention of biofilm formation by microorganisms.
